# EUROPEAN PATENT APPLICATION

(11) **EP 2 359 835 A1**
(43) Date of publication of application: **24.08.2011**
(21) Application number: 09820568.5
(22) Date of filing: 13.10.2009
(51) Int. Cl.: A61K 31/785, A61K 47/24, A61P 31/14, A61P 31/16, A61P 31/18, A61P 31/22, A61P 43/00

(54) **VIRAL INFECTION THERAPEUTIC DRUG CONTAINING POLYALKYLENEIMINE**

(30) Priority: 14.10.2008 JP 2008265560; 18.02.2009 JP 2009035803
(71) Applicant: Nippon Shokubai Co., Ltd., Osaka-shi, Osaka 541-0043 (JP)
(72) Inventor: HAYASHI Kyoko, Imizu-shi Toyama 939-0341 (JP); MAITANI Yoshie, Tokyo 158-0091 (JP); HASEGAWA Hiroaki, Suita-shi Osaka 564-8512 (JP); KAI Takashi, Tokyo 100-0011 (JP)
(74) Representative: Denison, Christopher Marcus
(86) International application number: PCT/JP2009/067713
(87) International publication number: WO 2010/044390

(57) **Abstract**

An object is to provide a therapeutic agent for viral infections that is less likely to give rise to drug-resistant virus owing to its different mechanism of action from that of previously available anti-viral drugs. When administered to the living body, polyalkyleneimine having a certain molecular weight adsorbs to the viruses invaded the living body to prevent them from adsorbing to and invading cells, whereby inactivating the viruses and thus treating or preventing viral infections.

## Description

### Technical Field

The present invention relates to a therapeutic agent for viral infections containing polyalkyleneimine.

### Background Art

Recently, viral infections caused by various viruses have become a big problem. For example, herpes simplex virus type 2 (HSV-2) is a pathogen that latently resides in the host after initial infection, which, however, later causes recurrent genital herpes. The prevalence rate of HSV-2 is high, and with drug therapies available today, an individual infected with this virus is deemed to have no choice but to remain latently infected for life. Further, recently, HSV-2 is gaining attention also as a factor for spreading infections and stimulating development of human immunodeficiency virus (HIV) (for example, see Non-Patent Document 1). Thus, drug therapy for HSV-2 infections is an important issue also from the viewpoint of anti-AIDS measures.

Presently as a therapeutic agent for herpes, nucleic acid analogs such as acyclovir and vidarabine, both of which are anti-viral drugs, are known. However, because these anti-viral drugs enter virus-infected cells to block viral proliferation, it is highly likely that the viruses mutate to acquire drug resistance so that they get around the effect of the drug. Thus, when long-term therapy is given, the emergence of drug-resistant virus needs to be watched. In view of the above circumstance, a therapeutic agent for viral infections having a different mechanism of action from that of previously available therapeutic agents is demanded.

From the viewpoint of the prevention of the emergence of drug-resistant virus, not a conventional therapeutic agent for viral infections that enters virus-infected cells but a therapeutic agent capable of inactivating the viruses before they infect the host cells is desired. As a method for inactivating viruses before they infect the host cells *in vitro,* for example, the method described in Patent Document 1 is known.

The invention described in Patent Document 1 relates to the prevention of a viral infection by addition of a virus-inactivating agent mainly composed of polyethyleneimine having an average molecular weight ranging from 500 to 8,000 to a sample composed of, for example, blood or a body fluid, whereby the virus contained in the sample is inactivated without affecting various test measurement values. However, polyethyleneimine is known to be highly toxic to the living body. Although the toxicity is not a big concern when no living cells are used, in the assessment systems involving living cells, particularly in the field of medicine, the toxicity is a big problem in the development of a novel therapeutic agent. For these reasons, very limited studies have been conducted to see how highly-toxic polyethyleneimine acts when administered to the living body, particularly, whether it can be used as a medicine or not.

Patent Document 2 is one of the few examples of administration of polyethyleneimine to the living body. Patent Document 2 describes that intraperitoneal administration of diluted linear polyethyleneimine of a molecular weight of 87,000 having a hydrophobic group such as a C18 acyl group introduced therein in the mouse led to the observation of immunostimulatory effects such as induction of interferon y. However, in Examples of Patent Document 2, only intraperitoneal administration of linear polyethyleneimine having a specific molecular weight and a specific hydrophobic group in the mouse is demonstrated. While polyethyleneimine is a polymer that may have various structures and molecular weights, no experiments on how polyethyleneimine other than the one disclosed in Patent Document 2, for example, polyalkyleneimine having different molecular weights, branched polyalkyleneimine, or unmodified polyalkyleneimine acts when administered to the living body has been known so far. Further, no case in which polyalkyleneimine is used as an external medicine has been known so far. Moreover, the direct action of polyalkyleneimine on viruses in the living body has not been known yet either.
Patent Document 1: JP Patent Publication (Kokai) No. 6-80520 A (1994)
Patent Document 2: JP Patent Publication (Kohyo) No. 2004-520328 A
Non-Patent Document 1: Wald et al., J. Infect. Dis., 185, p. 45 to 52, 2002

### Summary of Invention

### Problems to be solved by the invention

An object of the present invention is to provide a therapeutic agent for viral infections that is less likely to give rise to drug-resistant virus owing to its different mechanism of action from that of previously available anti-viral drugs.

### Means for solving the problems

As a result of a research, we have found that, when administered to the living body, polyalkyleneimine having a certain molecular weight adsorbs to the viruses invaded the living body to prevent them from adsorbing to the cell membrane and from invading the cells, whereby inactivating the viruses, and thus exerting therapeutic or preventive effects on viral infections.

Thus, the present invention encompasses the following inventions:

(1) A pharmaceutical composition for treating or preventing a viral infection, comprising polyalkyleneimine having a weight average molecular weight ranging from 300 to 400,000.

(2) The pharmaceutical composition according to (1), wherein the pharmaceutical composition is an external pharmaceutical composition comprising 20 mg/ml or less of the polyalkyleneimine.

(3) The pharmaceutical composition according to (1) or (2), wherein the polyalkyleneimine is linear or branched polyethyleneimine.

(4) The pharmaceutical composition according to any of (1) to (3), further comprising a lipid particle.

(5) The pharmaceutical composition according to any of (1) to (4), which is for treating or preventing an infection caused by an enveloped virus.

(6) The pharmaceutical composition according to any of (1) to (4), which is for treating or preventing a herpes virus infection.

(7) A virus-inactivating agent for administration to a living body, comprising polyalkyleneimine.

(8) An inhibitor of viral adsorption to a cell, comprising polyalkyleneimine.

(9) An inhibitor of viral invasion of a cell, comprising polyalkyleneimine.

(10) A method for treating or preventing a viral infection, comprising administering polyalkyleneimine having a weight average molecular weight ranging from 300 to 400,000.

(11) The method according to (10), comprising administering an external pharmaceutical composition comprising 20 mg/ml or less of the polyalkyleneimine.

(12) The method according to (10) or (11), wherein the polyalkyleneimine is linear or branched polyethyleneimine.

(13) The method according to any of (10) to (12), wherein polyalkyleneimine is administered with a lipid particle.

(14) The method according to any of (10) to (13), wherein the viral infection is an infection caused by an enveloped virus.

(15) The method according to any of (10) to (13), wherein the viral infection is a herpes virus infection.

(16) A method for inactivating a virus, comprising administering polyalkyleneimine to a living body.

(17) A method for inhibiting viral adsorption to a cell, comprising administering polyalkyleneimine.

(18) A method for inhibiting viral invasion of a cell, comprising administering polyalkyleneimine.

(19) Use of polyalkyleneimine having a weight average molecular weight ranging from 300 to 400,000 for the production of a pharmaceutical composition for treating or preventing a viral infection.

(20) The use according to (19), wherein the pharmaceutical composition is an external pharmaceutical composition comprising 20 mg/ml or less of the polyalkyleneimine.

(21) The use according to (19) or (20), wherein the polyalkyleneimine is linear or branched polyethyleneimine.

(22) The use according to any of (19) to (21), wherein the pharmaceutical composition comprises a lipid particle.

(23) The use according to any of (19) to (22), wherein the pharmaceutical composition is for treating or preventing an infection caused by an enveloped virus.

(24) The use according to any of (19) to (22), wherein the pharmaceutical composition is for treating or preventing a herpes virus infection.

(25) Use of polyalkyleneimine for the production of a virus-inactivating agent for administration to a living body.

(26) Use of polyalkyleneimine for the production of an inhibitor of viral adsorption to a cell.

(27) Use of polyalkyleneimine for the production of an inhibitor of viral invasion of a cell.

### Advantageous effects of invention

According to the present invention, a relatively inexpensive therapeutic agent for viral infections that is less likely to give rise to drug-resistant virus can be provided. Also, a non-virus type-specific therapeutic agent for viral infections can be provided.

### Brief Description of Drawings

[Figure 1] Figure 1 is a graph showing the time courses of disease development in HSV-2-infected mice.
[Figure 2] Figure 2 is a graph showing the changes in the body weight of HSV-2-infected mice.
[Figure 3] Figure 3 is a graph showing the results of a study comparing the effect of administration of polyethyleneimine versus acyclovir in HSV-2-infected mice.

The present specification encompasses the contents described in the specification, Claims, and the drawings of JP Patent Application Nos. 2008-265560 and 2009-035803, based on which the present application claims priority.

### Embodiments of the Invention

Polyalkyleneimine used in the present invention refers to a polymer obtained by polymerizing alkyleneimine containing 2 to 8 carbon atoms such as ethyleneimine, propyleneimine, butyleneimine, dimethylethyleneimine, pentyleneimine, hexyleneimine, heptyleneimine, and octyleneimine, particularly alkyleneimine containing 2 to 4 carbon atoms, by an ordinary method and a polymer obtained by chemically modifying those polymers by reacting with various compounds. Among the above polyalkyleneimine, polyethyleneimine (PEI) is particularly preferable in the present invention. Polyethyleneimine is a polymer that can be synthesized by, for example, a method of subjecting ethyleneimine to ring-opening polymerization using carbon dioxide, hydrochloric acid, hydrobromic acid, p-toluenesulfonic acid, aluminum chloride, boron trifluoride, or the like as a catalyst. Polyethyleneimine is widely utilized in various fields of, for example, adhesion, coating, and lamination. Specific examples of polyethyleneimine that can be used in the present invention include Epomin (registered trademark) commercially available from Nippon Shokubai Co., Ltd. (type: SP-003, SP-006, SP-012, SP-018, SP-200, and P-1000), polyethyleneimine commercially available from Wako Pure Chemical Industries, Ltd. (distributor's code 161-17831 (an average molecular weight of approximately 600), distributor's code 167-17811 (an average molecular weight of approximately 1,800), and distributor's code 164-17821 (an average molecular weight of approximately 10,000)), polyethyleneimine commercially available from Polysciences, Inc. (catalog No. 23966-2 (linear, MW 25,000)), and Lupasol (registered trademark) commercially available from BASF Japan Ltd. (type: Lupasol FG, Lupasol G20, Lupasol G35, and Lupasol PR8515).

Linear or branched polyalkyleneimine of various molecular weights, including polyethyleneimine, can be synthesized depending on the selection of the production method. For example, linear polyethyleneimine can be represented by the formula:

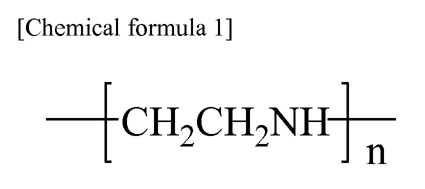

wherein, n ranges from 7 to 10000. Also, for example, branched polyethyleneimine can be represented by the following formula.

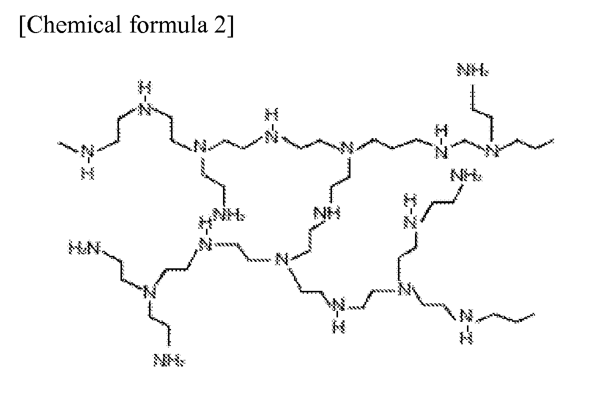

The degree of branching of polyalkyleneimine including polyethyleneimine can be expressed as the abundance ratio of a primary amino group, a secondary amino group, and a tertiary amino group present in the molecular framework. When the polyalkyleneimine of the present invention has a branched structure, no particular limitation is imposed on the ratio of each amino group; however, for example, polyalkyleneimine in which a primary amino group, a secondary amino group, and a tertiary amino group accounts for 25 to 45 mole%, 35 to 50 mole%, and 20 to 35 mole%, respectively, to the total amino group is preferably used. Particularly, polyalkyleneimine in which a primary amino group, a secondary amino group, and a tertiary amino group accounts for 30 to 40 mole%, 30 to 40 mole%, and 25 to 35 mole%, especially, 35 mole%, 35 mole%, and 30 mole%, respectively, to the total amino group is preferably used.

While polyalkyleneimine can have various structures depending on the production method, the polyalkyleneimine of the present invention may be either linear or branched. Also, although polyalkyleneimine can have various molecular weights, the weight average molecular weight of the polyalkyleneimine used in the present invention ranges from 300 to 400,000. Particularly, the weight average molecular weight ranges preferably from 300 to 30,000, more preferably from 2,000 to 30,000, and even more preferably from 2,000 to 25,000. The weight average molecular weight of the polyalkyleneimine used in the present invention most preferably ranges from 2,000 to 5,000. It is to be noted that the weight average molecular weight referred to herein is a value measured by gel permeation chromatography (GPC), using water soluble sugars (such as maltotriose and maltohexaose manufactured by Wako Pure Chemical Industries, Ltd. and pullulan P-82 manufactured by Showa Denko K.K.) as a standard reference material.

The polyalkyleneimine used in the present invention may be unmodified polyalkyleneimine or polyalkyleneimine chemically modified through reactions with various compounds, namely polyalkyleneimine having a substituent introduced to the nitrogen atom. Polyalkyleneimine can be modified by, for example, reacting it with aldehyde, ketone, alkyl halide, isocyanate, thioisocyanate, alkene, alkyne, a vinyl compound (such as acrylonitrile), an epoxy compound (such as epichlorohydrin), cyanamide, guanidine, urea, an organic acid (such as a fatty acid), acid anhydride, and acyl halide. Also, examples of the substituent to be introduced to the nitrogen atom in polyalkyleneimine by modification include C₁₋₆ alkyl, C₁-₆ hydroxyalkyl, C₁₋₆ alkenyl, acyl, and amide (these groups may further be substituted with hydroxyl, halo, cyano, nitro, acyl, amide, sulfonyl, sulfinyl, C₁₋₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, phenyl, benzyl, C₃₋₆ cycloalkyl, C₃₋₆ cycloalkenyl, and a 3 to 6-membered saturated, partially-unsaturated, or aromatic hetero ring). Examples of the reaction of polyalkyleneimine with various compounds are as shown in the following formulas.

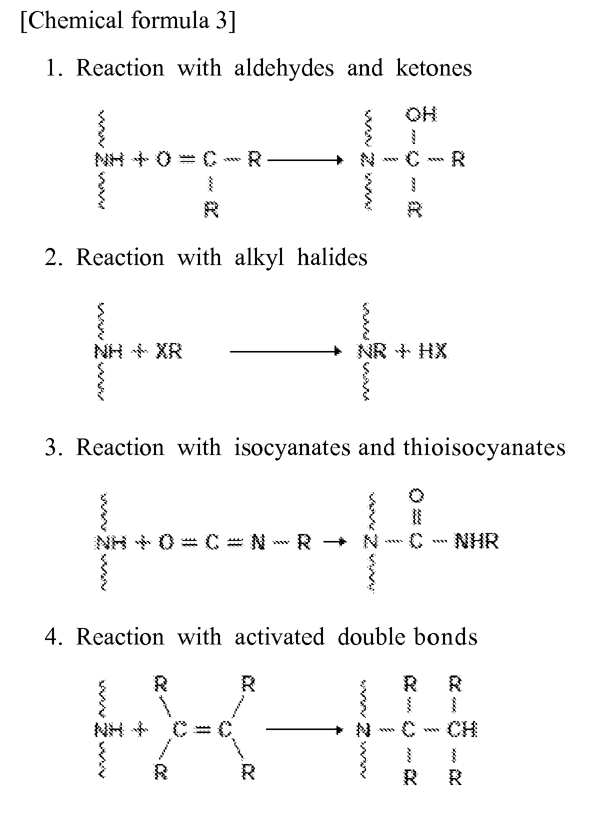

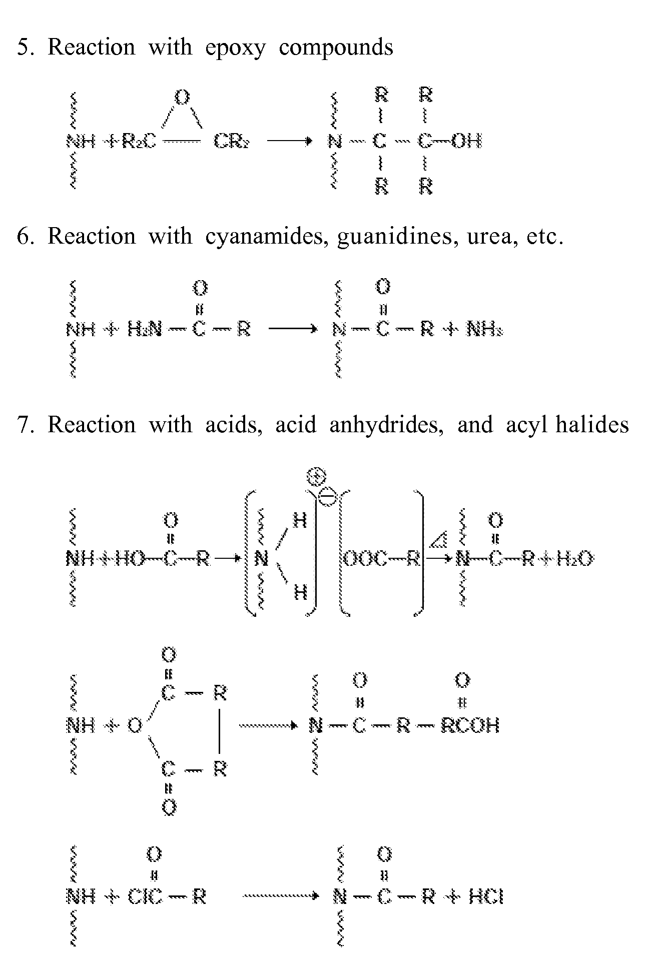

wherein, R is a substituent selected from hydroxyl, halo, cyano, nitro, acyl, amide, sulfonyl, sulfinyl, C₁₋₆ alkyl, C₁₋₆ haloalkyl, C₂₋₆ alkenyl, C₂₋₆ haloalkenyl, C₂₋₆ alkynyl, C₂₋₆ haloalkynyl, C₁₋₆ alkoxy, C₁₋₆ haloalkoxy, phenyl, benzyl, C₃₋₆ cycloalkyl, C₃₋₆ cycloalkenyl, and a 3 to 6-membered saturated, partially-unsaturated, or aromatic hetero ring.
Specific examples of modified polyalkyleneimine include polyalkyleneimine ethoxylate, particularly, polyethyleneimine ethoxylate. Polyalkyleneimine ethoxylate is a polymer having a structure in which a polyethylene glycol chain is bound to the nitrogen atom in the polyalkyleneimine. Polyalkyleneimine ethoxylate can be synthesized by, for example, reacting an epoxy compound with polyalkyleneimine. More specifically, for example, polyethyleneimine ethoxylate can be synthesized by feeding ethylene oxide to liquid polyethyleneimine under nitrogen atmosphere under conditions of high temperature, high pressure, and the presence of a catalyst to add ethylene oxide to the polyethyleneimine using the activated hydrogen contained in the amino group of the polyethyleneimine as a base point. Specific polyalkyleneimine ethoxylate that can be used in the present invention is preferably polyethyleneimine ethoxylate having a weight average molecular weight of approximately 2,000 to 15,000, preferably 4,000 to 12,000, and particularly approximately 5,500 to 10,000, and having a hydroxyl value of 50 to 500 mg KOH/g, preferably 70 to 400 mg KOH/g, as measured by the phthalic anhydride method. Among the above polyethyleneimine ethoxylate, polyethyleneimine ethoxylate having a dendrimer structure having polyethyleneimine in its center is particularly preferable. Examples of polyethyleneimine ethoxylate that can be used include one prepared by reacting ethylene oxide with Epomin SP-006 or SP-018 manufactured by Nippon Shokubai Co., Ltd.

Polyalkyleneimine is an extremely strongly cationic polymer. In contrast, proteins present on the viral surface responsible for binding to the host cells are known to be negatively charged. Thus, when polyalkyleneimine is administered to the environment in which viruses are present, the polyalkyleneimine electrostatically adsorbs to the viral surface, whereby preventing them from adsorbing to the cell membrane of host cells and from invading the cells, and thus inactivating the viruses. That is, polyalkyleneimine directly acts on viruses in the living body to inactivate them, and is particularly effective for, for example, the viral infection that cannot be treated or prevented by an immunostimulant that enhances the immune function of the living body. In view of the above, the present invention relates to not only a pharmaceutical composition for treating or preventing the viral infection but also a virus-inactivating agent for administration to the living body having a mechanism of action such that, when administered to the living body, it adsorbs to the viruses invaded the living body to block the activity of the viruses. Further, the present invention also relates to an inhibitor of viral adsorption to the cells. This inhibitor adsorbs to the viruses that have invaded the living body but not yet adsorbed to the cells, whereby inhibiting the adsorbability of the viruses to the cells. Further, the present invention also relates to an inhibitor of viral invasion of the cells, which inhibits the cell invasion ability of the viruses already adsorbed to the cells.

According to the aforementioned mechanism of action, the polyalkyleneimine of the present invention attains a non-specific antiviral action (an action of preventing and treating viral diseases through inhibition of viral proliferation). That is, the polyalkyleneimine has an anti-viral action on various viruses such as viruses belonging to *Herpesviridae* such as herpes simplex virus type 1 (HSV-1), herpes simplex virus type 2 (HSV-2), varicella-zoster virus (VZV), and cytomegalovirus (CMV) (collectively called the herpes virus), viruses belonging to *Orthomyxoviridae* such as influenza A virus, influenza B virus, and influenza C virus (collectively called the influenza virus), viruses belonging to *Retroviridae* such as human immunodeficiency virus (HIV), viruses belonging to *Paramyxoviridae* such as measles virus and mumps virus, viruses belonging to *Picornaviridae* such as poliovirus, rhinovirus, and hepatitis A virus, viruses belonging to *Hepadnaviridae* such as hepatitis B virus, viruses belonging to *Flaviviridae* such as hepatitis C virus, Japanese encephalitis virus, and west Nile virus, viruses belonging to *Adenoviridae* such as human adenovirus C, viruses belonging to *Coronaviridae* such as corona virus and SARS virus, viruses belonging to *Togaviridae* such as rubella virus, viruses belonging to *Rhabdoviridae* such as rabies virus and vesicular stomatitis alagoas virus, viruses belonging to *Filoviridae* such as Ebola virus, and viruses belonging to *Papovaviridae* such as human papillomavirus (HPV). The polyalkyleneimine of the present invention is effective for the treatment of infectious disease caused by the infection of these viruses. For example, the polyalkyleneimine of the present invention exerts an excellent anti-viral action on herpes virus, influenza virus, human immunodeficiency virus, measles virus, poliovirus, and rhinovirus, and thus is effective for the treatment of infectious disease caused by the infection of these viruses. Among these viruses, the polyalkyleneimine of the present invention exerts an excellent anti-viral action on enveloped viruses belonging to *Herpesviridae, Orthomyxoviridae, Retroviridae, Paramyxoviridae, Hepadnaviridae, Flaviviridae, Coronaviridae, Togaviridae, Rhabdoviridae,* and the like, particularly, enveloped viruses such as herpes virus, measles virus, influenza virus, corona virus, and human immunodeficiency virus, and thus is effective for the treatment of infectious disease caused by the infection of these viruses. Also, branched polyethyleneimine having a weight average molecular weight of approximately 2,310 to 4,600 or linear polyethyleneimine having a weight average molecular weight of approximately 25,000 is particularly effective for herpes virus; branched polyethyleneimine having a weight average molecular weight of approximately 2,310 is particularly effective for measles virus; polyethyleneimine ethoxylate having a weight average molecular weight of approximately 10,000 is particularly effective for influenza virus; and branched polyethyleneimine having a weight average molecular weight of approximately 1,470 to 321,200 or polyethyleneimine ethoxylate having a weight average molecular weight of approximately 10,000 is particularly effective for corona virus.

Particularly, the polyalkyleneimine of the present invention is effective for the prevention or treatment of infectious disease of herpes virus caused by the infection of herpes virus such as herpes labialis, herpetic stomatitis, herpes keratitis, genital herpes, neonatal herpes, varicella, and herpes zoster. Among these diseases, the polyalkyleneimine of the present invention is particularly effective for the prevention or treatment of genital herpes caused by the infection of herpes simplex virus type 2. Further, the polyalkyleneimine of the present invention is also effective for viruses that have acquired resistance to conventional anti-viral drugs such as acyclovir. Moreover, besides these diseases, the polyalkyleneimine of the present invention is also effective for the prevention or treatment of influenza, AIDS, measles, mumps, polio, common cold syndrome, hepatitis, Japanese encephalitis, West Nile fever, SARS, rubella, rabies, Ebola hemorrhagic fever, condylomata acuminate, wart, and uterine cervix cancer.

The polyalkyleneimine of the present invention can be administered as a pharmaceutical composition by itself or as a mixture with various pharmaceutically acceptable formulation aids. Generally, depending on the purpose, the polyalkyleneimine of the present invention can be administered in a dosage form suitable for application such as oral administration, intravenous administration, local administration, percutaneous or transmucosal administration. Examples of various dosage forms include an internal medicine for oral administration, an injection agent for parenteral administration including intravenous administration, or an external medicine for external application including percutaneous or transmucosal administration (a dermatologic agent, a suppository, an eye drop, a nasal drop, an ear drop, an oral disintegrant, an inhalant, or the like). It is to be noted that the external medicine means all of the drugs that are directly applied to a human body except an internal medicine and an injection agent. Also, external application means direct administration of a drug to the eye, the nose, the ear, the oral cavity, the anus, the skin, or the mucosa, including percutaneous or transmucosal administration.

The internal medicine includes a pharmaceutically acceptable aqueous medicine, suspension, emulsion, syrup, elixir, and the like. These liquid medicines can be obtained by dissolving, suspending, or emulsifying an active substance containing polyalkyleneimine into a diluent generally used (for example, purified water, ethanol, or a mixture of these liquids). Further, the liquid medicines may contain a humectant, a suspending agent, an emulsifying agent, a sweetener, a flavoring agent, an aromatic agent, a preservative, a buffer, or the like.

Examples of the dosage form of the external medicine include an ointment, a gel, a cream, a poultice, a cataplasm, a liniment, a nebulizing agent, an inhalant, a spray, an aerosol, an eye drop, and a nasal drop.

The ointment is produced according to publicly-known or generally-used formulations. For example, the ointment is prepared by triturating or dissolving an active substance containing polyalkyleneimine in a base. The base of the ointment is selected from publicly-known or generally-used bases. For example, a base selected from higher fatty acids or higher fatty acid esters (for example, adipic acid, myristic acid, palmitic acid, stearic acid, oleic acid, adipic acid ester, myristic acid ester, palmitic acid ester, stearic acid ester, or oleic acid ester), waxes (for example, beeswax, whale wax, or ceresin), surfactants (for example, polyoxyethylene alkyl ether phosphate), higher alcohols (for example, cetanol, stearyl alcohol, or cetostearyl alcohol), silicone oil (for example, dimethyl polysiloxane), hydrocarbons (for example, hydrophilic petrolatum, white petrolatum, purified lanolin, or liquid paraffin), glycols (for example, ethylene glycol, diethylene glycol, propylene glycol, polyethylene glycol, or macrogol), vegetable oil (for example, castor oil, olive oil, sesame oil, or turpentine oil), animal oil (for example, mink oil, egg-yolk oil, squalane, or squalene), water, absorption promoters, and rash-preventives is used alone, or a mixture of two or more of these bases is used. The ointment may further contain humectants, preservatives, stabilizers, antioxidants, scenting agents, or the like.

The gel is produced according to publicly-known or generally-used formulations. For example, the gel is prepared by dissolving an active substance containing polyalkyleneimine in a base. The base of the gel is selected from publicly-known or generally-used bases. For example, a base selected from lower alcohols (for example, ethanol or isopropyl alcohol), gelling agents (for example, carboxymethyl cellulose, hydroxyethyl cellulose, hydroxypropyl cellulose, or ethyl cellulose), neutralizers (for example, triethanolamine or diisopropanolamine), surfactants (for example, polyethylene glycol monostearate), gums, water, absorption promoters, and rash-preventives is used alone, or a mixture of two or more of these bases is used. The gel may further contain preservatives, antioxidants, scenting agents, or the like.

The cream is produced according to publicly-known or generally-used formulations. For example, the cream is produced by dissolving or emulsifying an active substance containing polyalkyleneimine in a base. The base of the cream is selected from publicly-known or generally-used bases. For example, a base selected from higher fatty acid esters, lower alcohols, hydrocarbons, polyhydric alcohols (for example, propylene glycol or 1,3-butylene glycol), higher alcohols (for example, 2-hexyl decanol or cetanol), emulsifiers (for example, polyoxyethylene alkyl ethers or fatty acid esters), water, absorption promoters, and rash-preventives is used alone, or a mixture of two or more of these bases is used. The cream may further contain preservatives, antioxidants, scenting agents, or the like.

The poultice is produced according to publicly-known or generally-used formulations. For example, the poultice is produced by dissolving an active substance containing polyalkyleneimine in a base, and then spreading the resulting combined mixture over a base material. The base of the poultice is selected from publicly-known or generally-used bases. For example, a base selected from viscosity enhancers (for example, polyacrylic acid, polyvinylpyrrolidone, gum arabic, starch, gelatin, or methyl cellulose), humectants (for example, urea, glycerin, or propylene glycol), fillers (for example, kaolin, zinc oxide, talc, calcium, or magnesium), water, solubilizing aids, tackifiers, and rash-preventives is used alone, or a mixture of two or more of these bases is used. The poultice may further contain preservatives, antioxidants, scenting agents, or the like.

The cataplasm is produced according to publicly-known or generally-used formulations. For example, the cataplasm is produced by dissolving an active substance containing polyalkyleneimine in a base, and then spreading the resulting mixture over a base material. The base of the cataplasm is selected from publicly-known or generally-used bases. For example, a base selected from polymer bases, oils, fats, higher fatty acids, tackifiers, and rash-preventives is used alone, or a mixture of two or more of these bases is used. The cataplasm may further contain preservatives, antioxidants, scenting agents, or the like.

The liniment is produced according to publicly-known or generally-used formulations. For example, the liniment is produced by dissolving, suspending, or emulsifying an active substance containing polyalkyleneimine in one or more of bases selected from water, alcohols (for example, ethanol and polyethylene glycol), higher fatty acids, glycerin, soap, emulsifiers, suspending agents, and the like. The liniment may further contain preservatives, antioxidants, scenting agents, or the like.

The nebulizing agent, the inhalant, and the spray may contain, besides generally-used diluents, stabilizers such as sodium bisulfite and buffers imparting isotonicity, for example, isotonic agents such as sodium chloride, sodium citrate, or citric acid.

The injection agent encompasses all kinds of injection agents including drip infusion fluids. For example, the injection agent encompasses injection agents for intramuscular, subcutaneous, intracutaneous, intra-arterial, intravenous, intraperitoneal, and intraspinal administration and drip infusion fluids for intravenous administration.

The injection agent encompasses solutions, suspensions, emulsions, and solid injection agents that are dissolved or suspended in a solvent before use. The injection agent is prepared by dissolving, suspending, or emulsifying an active substance containing polyalkyleneimine in a solvent. Examples of the solvent include distilled water for injection, physiological saline, vegetable oil, propylene glycol, polyethylene glycol, alcohols such as ethanol, and a combination of these solvents. The injection agent may further contain stabilizers, solubilizing aids (for example, glutamic acid, aspartic acid, or Polysorbate 80 (registered trademark)), suspending agents, emulsifiers, soothing agents, buffers, and preservatives. The injection agent is sterilized in the final production process or prepared by the aseptic technique. Alternatively, a sterile solid agent, for example, a freeze dried agent can be produced, and an injection agent prepared with this solid agent can be sterilized before use or this solid agent can be dissolved in sterile distilled water for injection or other solvents.

The eye drop encompasses eye drop liquids, eye drop suspensions, eye drop emulsions, eye drop liquids prepared by dissolving before use, and eye ointments. The eye drop is produced according to publicly-known methods. For example, the eye drop is prepared by dissolving, suspending, or emulsifying an active substance containing polyalkyleneimine in a solvent. Examples of the solvent for the eye drop include sterilized purified water, physiological saline, other aqueous solvents or non-aqueous solvents for injection (for example, vegetable oil), and a combination of these solvents. The eye drop may contain appropriately selected substances such as isotonic agents (for example, sodium chloride and concentrated glycerin), buffers (for example, sodium phosphate and sodium acetate), surfactants (for example, Polysorbate 80 (trade name), polyoxyl 40 stearate, for example, polyoxyethylene hydrogenated castor oil), stabilizers (for example, sodium citrate or sodium edetate), preservatives (for example, benzalkonium chloride or paraben) as needed. The eye drop is sterilized in the final production process or prepared by the aseptic technique. Alternatively, a sterile solid agent, for example, a freeze dried agent can be produced, and an eye drop prepared with this solid agent can be sterilized before use or this solid agent can be dissolved in sterile distilled water or other solvents.

The inhalant encompasses aerosols, powder for inhalation, or liquids for inhalation. The liquid for inhalation may be formulated to be dissolved or suspended in water or other appropriate media before use. The inhalant is produced according to publicly-known methods. For example, the liquid for inhalation may be prepared with appropriately selected substances such as preservatives (for example, benzalkonium chloride or paraben), colorants, buffers (for example, sodium phosphate or sodium acetate), isotonic agents (for example, sodium chloride or concentrated glycerin), tackifiers (for example, carboxy vinyl polymers), and absorption promoters as needed, and the powder for inhalation may be prepared with appropriately selected substances such as lubricants (for example, stearic acid and a salt thereof), binders (for example, starch or dextrin), excipients (for example, lactose or cellulose), colorants, preservatives (for example, benzalkonium chloride or paraben), absorption promoters as needed. When administering the liquid for inhalation, a sprayer (for example, an atomizer or a nebulizer) is normally used, while when administering the powder for inhalation, a device for inhalation of a powdered drug is normally used.

Examples of the compositions for external application other than the ones given above include suppositories for intrarectal administration and pessaries for vaginal administration, both of which contain an active substance containing polyalkyleneimine and are formulated according to ordinary methods.

The pharmaceutical composition containing the polyalkyleneimine of the present invention is preferably externally applied locally to the site affected by the viral infection, especially via percutaneous or transmucosal administration. Further, the polyalkyleneimine is preferably externally applied in a concentration of 20 mg/ml or less, particularly preferably in a concentration of 0.004 mg/ml to 20 mg/ml. Meanwhile, the dose for, for example a mammal, is preferably set to approximately 0.001 to 50 mg per kg body weight per day, based on the weight of polyalkyleneimine. In external application, as long as such a concentration or such a dose of polyalkyleneimine is administered, the toxicity of the polyalkyleneimine is suppressed, while its anti-viral action is maintained. That is, the toxicity of polyethyleneimine to the living body manifests as, for example, affecting the living body in such a way that it reduces the body weight and weakens an individual, ultimately leading to death; however, as long as the polyalkyleneimine is administered in such a concentration or in such a dose as described above, the viral infection of a polyalkyleneimine-administered animal can be cured without killing the animal. Also, the concentration of polyalkyleneimine for external application is preferably 10 mg/ml or less as the toxicity can be further suppressed.

The pharmaceutical composition containing the polyalkyleneimine of the present invention may be administered either before or after the viral infection; however, it exerts favorable effects when administered before or right after the viral infection. The pharmaceutical composition may be administered only one day or continuously for several days to a virus-infected animal. In external application, when the pharmaceutical composition is administered in a relatively highly-concentrated dosage form with a concentration of particularly approximately 10 mg/ml to 20 mg/ml, especially approximately 20 mg/ml, it exerts sufficient effects even when administered only one day. Also, when the pharmaceutical composition is administered in a relatively low-concentrated dosage form with a concentration of approximately 0.004 mg/ml to 10 mg/ml, particularly approximately 0.004 mg/ml to 1 mg/ml, and especially approximately 0.004 mg/ml to 0.4 mg/ml, it is preferably administered continuously for certain period of days, for example approximately for two weeks, or at least for three to seven days. The administration may be once or divided into two to three times or four to five times a day.

The toxicity of polyalkyleneimine can also be suppressed by modification of the polyalkyleneimine. For example, the toxicity to the living body can be suppressed by converting polyalkyleneimine into polyalkyleneimine ethoxylate, particularly polyethyleneimine ethoxylate.

The toxicity of polyalkyleneimine can also be suppressed by using polyalkyleneimine in combination with lipid particles. The lipid particle refers to a concept encompassing particles composed of lipid aggregate, particles having the surface covered by lipid and containing lipophilic components inside (micelle), liposomes, and the like. The lipid particle of the present invention is preferably in a form of any of polymeric micelle, emulsion, fine particle, nanoparticle, and liposome.

The polymeric micelle is a fine nanoparticle having a size of several tens of nanometers, which is produced by autonomous gathering of amphiphilic block copolymers composed of hydrophilic polymers and hydrophobic polymers dispersed in water. As the block copolymer, a PEG-polyamino acid or a derivative thereof, or the like is used. The emulsion refers to a dispersion system in which both the solute and the solvent are liquid. The fine particle refers to a spherical preparation having a particle diameter of approximately several µm, and the nanoparticle refers to the fine particle with a nanoscale diameter (normally, 1 µm or less).

The liposome is a closed vesicle composed of a phospholipid bilayer, containing water inside. No particular limitation is imposed on the type of liposome used in the present invention, and any type such as multilamellar vesicle (MLV) and small or large unilamellar vesicle (SUV, LUV) may be used. Also, the liposome may be prepared using any publicly known technique such as thin-film method, freeze-dry method, spray-dry method, reverse phase evaporation method, surfactant removal method, and ethanol injection method. Also, the particle diameter of the liposome thus obtained may be adjusted with an extruder, a high pressure homogenizer, an ultrasonic wave, and the like. Publicly-known various types of phospholipids may be used as the components of the liposomal membrane. For example, phosphatidylcholine (lecithin) derived from egg yolk, soybean, and other natural substances, phosphatidylethanolamine, phosphatidylinositol, phosphatidylserine, phosphatidic acid, oleic acid, ceramide, sphingomyelin, and hydrogenated products of these substances, and synthetically obtainable dipalmitoylphosphatidylcholine, distearoylphosphatidylcholine, dimyristoylphosphatidylcholine, dicetyl phosphate containing charged lipid, dihexadecyl phosphate, a dioctadecyldimethylammonium salt, stearylamine, and other artificial lipids, cationic lipids, and the like can be used alone or a mixture of these substances can be used. The liposome used in the present invention is particularly preferably a positively-charged liposome with the positively-charged surface. Such a positively-charged liposome can be prepared by mixing egg yolk lecithin, cholesterol, and stearylamine. Further, the liposome used in the present invention is also particularly preferably a neutral liposome composed of egg yolk lecithin and cholesterol, or a liposome obtained by coating such a neutral liposome with polyethylene glycol. As to the size of the liposome of the present invention, it has a particle diameter of preferably approximately 0.025 to 200 µm, more preferably particularly approximately 0.025 to 100 µm, and even more preferably especially approximately 0.025 to 10 µm.

While polyalkyleneimine can be used alone or in combination with liposomes, it can further be used in combination with other active substances. Examples of the active substance other than ones described above include publicly known anti-viral drugs such as acyclovir, valacyclovir, penciclovir, famciclovir, vidarabine, idoxuridine, sorivudine, brivudine, ganciclovir, valganciclovir hydrochloride, foscarnet sodium hydrate, oseltamivir, zanamivir, amantadine, rimantadine, zidovudine, didanosine, zalcitabine, sanilvudine, lamivudine, abacavir, tenofovir, emtricitabine, nevirapine, efavirenz, delavirdine, indinavir, saquinavir, lopinavir, ritonavir, nelfinavir, amprenavir, atazanavir, fosamprenavir, enfuvirtide, adefovir, entecavir, and ribavirin, and virucidal disinfectants such as iodine disinfectants including povidone-iodine and chlorhexidine. The liposome and other active substances may be contained in a preparation containing the polyalkyleneimine of the present invention, or, as a preparation separate from a preparation containing the polyalkyleneimine of the present invention, the liposome and other active substances may be administered simultaneously or sequentially with the preparation containing polyalkyleneimine.

### [Examples]

Hereinbelow, the present invention will be described in more detail with reference to Examples; however, the technical scope of the present invention is not limited to the following Examples.

### Example 1: The preventive effect of external application of polyethyleneimine (SP-006 and P25K) on the development of the disease in HSV-2-infected animals

An infection experiment was conducted on the anti-viral action of polyethyleneimine (PEI) on herpes simplex virus type 2 (HSV-2) in mice.

As the polyethyleneimine, Epomin (registered trademark) SP-006 manufactured by Nippon Shokubai Co., Ltd. (branched, a weight average molecular weight of 2,310 as measured by GPC) and polyethyleneimine manufactured by Polysciences, Inc. (catalog No. 23966-2, linear, a weight average molecular weight of 25,000: hereinbelow, this polyethyleneimine will be referred to as "P25K") were used. Two polyethyleneimines, namely SP-006 and P25K, were adjusted to pH 7.0 to 7.4 with 1N hydrochloric acid. Solutions of 20 mg/ml and 0.4 mg/ml (diluting a solution of 20 mg/ml 50-fold with phosphate-buffered physiological saline (PBS)) of SP-006 and solutions of 20 mg/ml and 0.004 mg/ml (diluting a solution of 20 mg/ml 5000-fold with phosphate-buffered physiological saline) of P25K were each used. Phosphate-buffered physiological saline (PBS) was used as a control.

Five to six-week-old female BALB/c mice were locally (genital) inoculated with HSV-2 in an amount of 20 µl (containing 1 × 10⁵ plaque forming unit (PFU) of virus) per mouse. SP-006, P25K, or phosphate-buffered physiological saline was initially administered one hour before the viral infection, and thereafter, three times daily (8:00 am, 1:00 pm, and 6:00 pm) for one week by application to the genital mucosa in a dose of 20 µl per administration. The results thus obtained are as shown in Table 1.

The BALB/c mice were inoculated with a lethal dose of HSV-2, and all 13 mice died within 10 days of the viral infection in the control group. Mice administered with solutions of 20 mg/ml of SP-006 and P25K three times on the day of infection were obviously weakened, thus administration was discontinued on the second day. Among three mice administered with a solution of 20 mg/ml of SP-006, only one mouse died on the first day of administration (weakening and death are considered to be caused by the toxicity of polyethyleneimine). In other administration groups, no toxicity was observed in mice (judged from changes in the body weight and appearance).

The amount of virus in the genital three days after the viral infection was measured by plaque assaying using a wash solution collected by washing the genital of the mice with PBS. As a result, compared to the control group, it was found drastically decreased even in the mice administered with solutions of 20 mg/ml of SP-006 and P25K only three times. Further, despite the administration of quite low doses, the viral proliferation-inhibitory effect was exhibited in the mice administered with a 50-fold diluted solution of SP-006 (0.4 mg/ml) and a 5000-fold diluted solution of P25K (0.004 mg/ml). Furthermore, in any polyethyleneimine administration group, a death-prevention effect and a survival period-prolonging effect were observed.

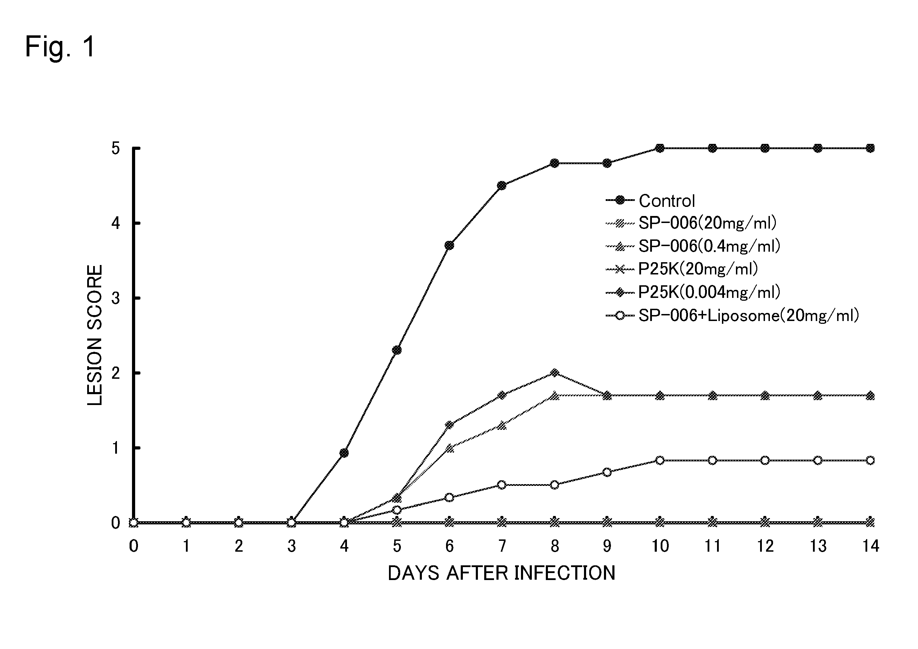

A BALB/c mouse inoculated with HSV-2 in its genital develops similar inflammation and oedema to those observed in human genital herpes. Based on these symptoms, the severity of the development of the disease was scored according to the following five stages: 0: asymptomatic, 1: mild genital inflammation, 2: moderate inflammation with oedema, 3: severe inflammation with exudate, 4: hind limb paralysis, and 5: death. The course of the development of genital herpes was expressed in the above score (an average obtained from each mouse) for two weeks after the viral infection, which were shown in Figure 1. In the control group, the mice began to develop the disease four days after the viral infection, which were aggravated with time. In contrast, all of the mice administered with solutions of 20 mg/ml of SP-006 and P25K three times lived asymptomatically. Although the mice administered with a low concentration of polyethyleneimine began to develop the disease five days after the infection, the severity of the disease was milder than that observed in the control group.

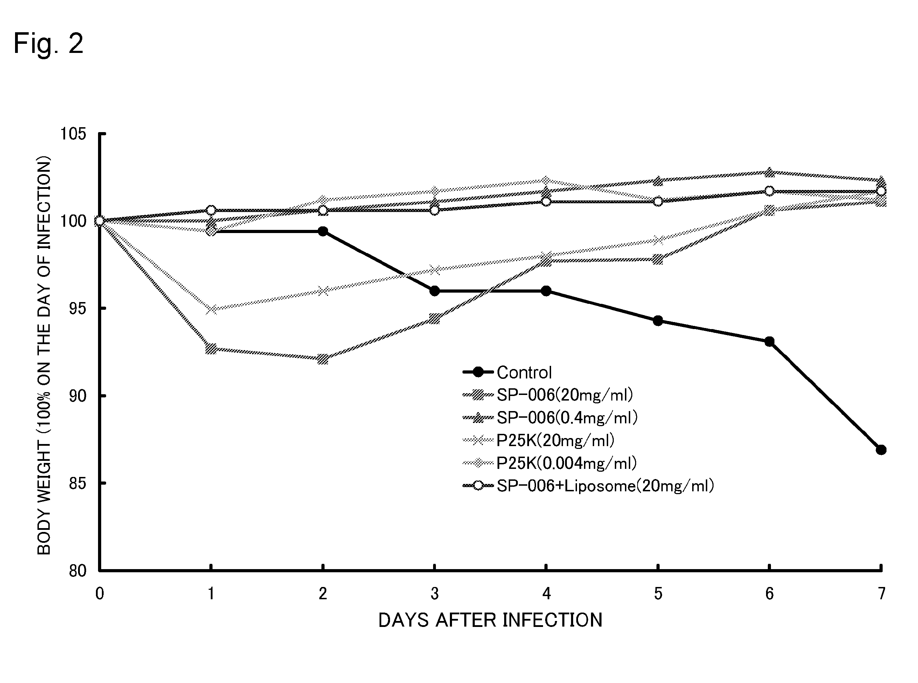

Figure 2 shows the changes in the average body weight of surviving mice with setting the day of infection at 100%. Although the body weight of the groups of mice administered with solutions of 20 mg/ml of SP-006 and P25K only three times was greatly decreased on the first day due to the toxicity, it is understood that, upon discontinuation of administration and healing of the viral infection, the mice recovered thereafter. The body weight of the groups of mice continuously administered with a solution of 0.4 mg/ml of SP-006 and a solution of 0.004 mg/ml of P25K almost did not change. Meanwhile, the body weight of the mice in the control group was gradually decreased due to the viral infection, leading to the death of the mice.

In view of the above, it is understood that, although a concentration of polyethyleneimine of approximately 20 mg/ml is slightly toxic to the mice and thus is not suitable for continuous administration, a few administrations exhibit a potent therapeutic effect on genital herpes. Further, it is understood that a low concentration of polyethyleneimine of 0.4 mg/ml or 0.004 mg/ml exerts a viral proliferation-inhibitory effect sufficient to prevent the development of the disease and death without exhibiting toxicity in mice.

**[Table 1]**

| The preventive effect of external application of polyethyleneimine on the development of the disease in HSV-2-infected animals | | | | | |
|---|---|---|---|---|---|
| Sample | PEI dose per administration | Number of mouse | Amount of virus 3 days after infection (×10² PFU/100µl) | Survival rate (%) | Days until death (median number of days) |
| Control (PBS) | - | 13 | 162±82 | 0/13 (0) | 6,6,6,6,6,7,7, 7,7,7,8,8,9 (7.0 ± 0.77) |
| SP-006* | 0.4 mg | 2 | 0+0 | 2/2 (100) | - |
| SP-006 | 8µg | 3 | 13±7.4 | 2/3 (67) | 9 (9.0 ± 0) |
| P25K* | 0.4 mg | 3 | 1.6 ± 2.8 | 3/3 (100) | - |
| P25K | 0.08µg | 3 | 50±61 | 2/3 (67) | 8 (8.0 ± 0) |
| SP-006 + liposome** | 0.4 mg | 6 | 4.2 ±7.3 | 5/6 (83) | 10 (10.0 ± 0) |

| | | | | | |
|---|---|---|---|---|---|
| * Due to the toxicity, administration was discontinued after three doses on the day of infection. ** Liposome is composed of egg yolk lecithin, cholesterol, and stearylamine (molar ratio of 5 : 5 : 4), containing the same mass as SP-006 (20 mg/ml). | | | | | |

### Example 2: The preventive effect of a complex of polyethyleneimine (SP-006) and liposomes on the development of the disease in HSV-2-infected animals

An aqueous solution of liposomes composed of egg yolk lecithin, cholesterol, and stearylamine (molar ratio of 5 : 5 : 4) (total lipid amount of 20 mg/ml) and SP-006 was adjusted to pH 7.0 to 7.4 with 1N hydrochloric acid. This polyethyleneimine-liposome complex contains SP-006 in a concentration of 20 mg/ml. The efficacy of this mixture in the BALB/c mouse model of genital herpes was evaluated. The experiment was performed similarly to the aforementioned Example 1.

The results thus obtained were shown in Table 1 and Figures 1 and 2 similarly to Example 1. Unlike the administration of a solution of 20 mg/ml of only SP-006, this complex did not exert any prominent toxicity in the mice even when continuously administered for one week. As a result of measurement of the amount of virus in the genital three days after infection, no virus was detected in four mice, and a trace amount of virus, which was 1/10 to 1/20 of the control group, was detected only in two mice, out of six. The death rate and the severity of the development of the disease were markedly reduced compared to the control group.

Based on the above results, it was understood that the toxicity of polyethyleneimine to the living body can be attenuated not only by diluting it but also by using it in combination with liposomes. Further, it was also understood that the natural anti-viral action of polyethyleneimine can be maintained even when it is used in combination with liposomes.

### Example 3: HSV-2 virus-inactivating action of polyethyleneimine

Whether HSV-2 virus was actually inactivated by polyethyleneimine was investigated. The experiment was performed in accordance with the following procedure.

(1) SP-006 or P25K was diluted to each concentration with a medium and PBS.
(2) HSV-2 (2 × 10² PFU/100 µl or 2 × 10⁴ PFU/100 µl) and the diluted solution of polyethyleneimine prepared in the aforementioned (1) were mixed at 1 : 1 and treated at 37°C for one hour.
(3) The amount of remaining virus in a sample prepared with 2 × 10² PFU/100 µl of virus was directly measured by plaque assaying. The amount of remaining virus in the sample prepared with 2 × 10⁴ PFU/100 µl of virus was measured in a sample diluted 100-fold with PBS by plaque assaying.
The results thus obtained were summarized in Table 2. The amount of remaining virus was increased in the diluted sample compared to the undiluted sample, showing that the virus-inactivating action was slightly attenuated. This result indicates that, although polyethyleneimine does not have a virucidal activity, it forms a loose bond (electrostatic bond) with the virus, whereby attaining a reversible preventive action on viral infection, namely, a virus-inactivating action.

**[Table 2]**

| HSV-2 virus-inactivating action of polyethyleneimine | | | |
|---|---|---|---|
| Sample | Concentration (µg/ml) | Not diluted | Diluted 100-fold |
| SP-006 | 9500 | 31 | 55 |
| | 950 | 35 | 72 |
| | 95 | 47 | 100 |
| | 9.5 | 98 | 98 |
| | 0.95 | 102 | 102 |
| | | | |
| P25K | 10000 | Toxic | 19 |
| | 1000 | Toxic | 66 |
| | 100 | 14 | 96 |
| | 10 | 22 | 106 |
| | 1 | 104 | 104 |

| | | | |
|---|---|---|---|
| * Values indicate the amount of virus (%) in each treatment with setting the amount of virus in the control group with no polyethyleneimine added at 100%. * "Toxic" indicates that the tested cells were killed by the toxicity of the sample. | | | |

### Comparative Example 1: Action of povidone iodine on HSV-2 virus

As a positive control, the virucidal activity of povidone iodine (Isodine (registered trademark) manufactured by Meiji Seika Kaisha, Ltd., containing 100 mg/ml of povidone iodine), which is a commercially available disinfectant exhibiting a potent virucidal activity, was investigated by a method similar to that of the aforementioned Example 3.

The results thus obtained were shown in Table 3. Povidone iodine behaved differently from polyethyleneimine. While diluted SP-006 and P25K exhibited attenuated virus-inactivating actions as observed in the increased amount of remaining virus, povidone iodine did not display big changes in its effect even when it was diluted. From these results, it is speculated that povidone iodine has an irreversible extinguishing action on the infectivity of viral particles, namely, a virucidal activity.

**[Table 3]**

| Action of povidone iodine on HSV-2 virus | | | |
|---|---|---|---|
| Sample | Concentration (µg/ml) | Not diluted | Diluted 100-fold |
| Povidone | 10000 | Toxic | 0 |
| iodine | 1000 | 0 | 0 |
| | 100 | 0 | 0 |
| | 10 | 0 | 0 |
| | 1 | 82 | 94 |

| | | | |
|---|---|---|---|
| * Values indicate the amount of virus (%) with sled by the toxicity of the sample.etting the amount of virus in the control group with no povidone iodine added at 100%. * "Toxic" indicates that the tested cells were kil | | | |

### Example 4: The inhibitory effect of polyethyleneimine on HSV-2 virus adsorption to the cells

Whether adsorption of HSV-2 virus to the host cells was inhibited by polyethyleneimine was investigated. The experiment was performed in accordance with the following procedure.

(1) Vero cells (24-well plate), a virus liquid (200 PFU/100 µl), and a twice-concentrated liquid of a polyethyleneimine liquid of a certain concentration (SP-006 or P25K) were cooled at 4°C for two hours.
(2) The virus liquid and the polyethyleneimine liquid were added, 100 µl each, to the Vero cells, followed by treatment at 4°C for one hour (at 4°C, although viruses adsorb to the cells, the subsequent viral invasion of the cells does not occur).
(3) After washing with ice-cooled phosphate-buffered physiological saline twice, the plate was overlaid with a medium containing no sample (containing 0.8% methyl cellulose).
(4) After three days, the number of plaques was counted.

The amount of virus adsorbed to the cells in the presence of the sample is shown in Table 4. SP-006 and P25K dose-dependently inhibited viral adsorption.

**[Table 4]**

| The inhibitory effect of polyethyleneimine on HSV-2 virus adsorption to the cells | | |
|---|---|---|
| Sample | Concentration (µg/ml) | Amount of virus adsorbed to host cells |
| SP-006 | 9500 | 27 |
| | 950 | 47 |
| | 95 | 74 |
| | 9.5 | 76 |
| | 0.95 | 97 |
| | | |
| P25K | 10000 | Toxic |
| | 1000 | Toxic |
| | 100 | 20 |
| | 10 | 36 |
| | 1 | 63 |

| | | |
|---|---|---|
| * Values indicate the amount of virus (%) in each treatment with setting the amount of virus in the control group with no polyethyleneimine added at 100%. * "Toxic" indicates that the tested cells were killed by the toxicity of the sample. | | |

### Example 5: The inhibitory effect of polyethyleneimine on HSV-2 virus invasion of the cells

Subsequently, the inhibitory effect of polyethyleneimine in the stage in which HSV-2 adsorbed to the host cells invades the cells was investigated. The experiment was performed in accordance with the following procedure.

(1) Vero cells (24-well plate) and a virus liquid (100 PFU/100 µl) were cooled at 4°C for two hours.
(2) The virus liquid (100 µl) was added to the Vero cells, followed by treatment at 4°C for one hour.
(3) After washing with ice-cooled phosphate-buffered physiological saline twice, a medium containing polyethyleneimine (SP-006 or P25K) was added, followed by culturing at 37°C (by leaving the plate at this temperature, viruses adsorbed to the cell initiate invasion).
(4) After 30 minutes or three hours, the plate was treated with a citrate buffer (pH 3.0) for one minute to inactivate the viruses that had not invaded yet.
(5) Immediately after that, the plate was overlaid with a medium (containing 0.8% methyl cellulose).
(6) After three days, the number of plaques was counted.
The amount of virus invaded the cells in the presence of the sample is shown in Table 5. SP-006 and P25K dose-dependently inhibited viral invasion. This effect was more potent than the viral adsorption-inhibitory effect. Also, the viral invasion-inhibitory effect was observed even at the concentration at which no virus inactivation was observed.

**[Table 5]**

| The inhibitory effect of polyethyleneimine on HSV-2 virus invasion of the cells | | | |
|---|---|---|---|
| Sample | Concentration (µg/ml) | Amount of virus invaded in 30 minutes | Amount of virus invaded in 3 hours |
| SP-006 | 9500 | 14 | 28 |
| | 950 | 23 | 26 |
| | 95 | 28 | 35 |
| | 9.5 | 37 | 40 |
| | 0.95 | 37 | 42 |
| | | | |
| P25K | 10000 | 7 | Toxic |
| | 1000 | 35 | Toxic |
| | 100 | 33 | 5 |
| | 10 | 33 | 33 |
| | 1 | 53 | 56 |

| | | | |
|---|---|---|---|
| * Values indicate the amount of virus (%) in each treatment with setting the amount of virus in the control group with no polyethyleneimine added at 100%. * "Toxic" indicates that the tested cells were killed by the toxicity of the sample. | | | |

### Comparative Example 2: The inhibitory effect of povidone iodine on HSV-2 virus adsorption to and invasion of the cells

The effect of povidone iodine on HSV-2 adsorption to the host cells was investigated. The experiment was performed similarly to the aforementioned Example 4. The amount of virus adsorbed to the cells in the presence of the sample as a result is shown in Table 6. Unlike polyethyleneimine, povidone iodine exhibited almost no dose-dependent adsorption-inhibitory effect.

**[Table 6]**

| The inhibitory effect of povidone iodine on HSV-2 virus adsorption to the cells | | |
|---|---|---|
| Sample | Concentration (µg/ml) | Amount of virus adsorbed to host cells |
| Povidone | 10000 | Toxic |
| iodine | 1000 | Toxic |
| | 100 | 69 |
| | 10 | 94 |
| | 1 | 97 |

| | | |
|---|---|---|
| * Values indicate the amount of virus (%) with setting the amount of virus in the control group with no povidone iodine added at 100%. * "Toxic" indicates that the tested cells were killed by the toxicity of the sample. | | |

The inhibitory effect of povidone iodine on HSV-2 virus invasion of the cells was investigated by a similar method to that of the aforementioned Example 5. The amount of virus invaded the cells in the presence of povidone iodine is shown in Table 7. Povidone iodine seemingly exhibits a fair invasion-inhibitory effect even at a low concentration. However, while polyethyleneimine exhibited an invasion-inhibitory effect even at a concentration at which no virus-inactivating action is exerted, povidone iodine inhibited invasion at a concentration at which a virucidal effect is exerted. Based on the above results, it is considered that the invasion-inhibitory effect of povidone iodine is produced as a result of its virucidal activity.

**[Table 7]**

| The inhibitory effect of povidone iodine on HSV-2 virus invasion of the cells | | | |
|---|---|---|---|
| Sample | Concentration (µg/ml) | Amount of virus invaded in 30 minutes | Amount of virus invaded in 3 hours |
| Povidone | 10000 | Toxic | Toxic |
| iodine | 1000 | Toxic | Toxic |
| | 100 | 7 | 9 |
| | 10 | 35 | 44 |
| | 1 | 47 | 63 |

| | | | |
|---|---|---|---|
| * Values indicate the amount of virus (%) with setting the amount of virus in the control group with no povidone iodine added at 100%. * "Toxic" indicates that the tested cells were killed by the toxicity of the sample. | | | |

### Comparative Example 3: The inhibitory effect of an anti-herpes drug, acyclovir, on HSV-2 virus adsorption to and invasion of the cells

The effect of acyclovir on HSV-2 adsorption to the host cells was investigated. The experiment was performed similarly to the aforementioned Example 4. The amount of virus adsorbed to the cells in the presence of acyclovir as a result is shown in Table 8. Unlike polyethyleneimine, acyclovir exhibited almost no dose-dependent adsorption-inhibitory effect.

**[Table 8]**

| The inhibitory effect of acyclovir on HSV-2 virus adsorption to the cells | | |
|---|---|---|
| Sample | Concentration (µg/ml) | Amount of virus adsorbed to host cells |
| Acyclovir | 10000 | Toxic |
| | 1000 | Toxic |
| | 100 | 76 |
| | 10 | 93 |
| | 1 | 101 |

| | | |
|---|---|---|
| * Values indicate the amount of virus (%) with setting the amount of virus in the control group with no acyclovir added at 100%. * "Toxic" indicates that the tested cells were killed by the toxicity of the sample. | | |

The inhibitory effect of acyclovir on HSV-2 virus invasion of the cells was investigated by a similar method to that of the aforementioned Example 5. The amount of virus invaded the cells in the presence of acyclovir is shown in Table 9. Acyclovir did not inhibit HSV-2 virus invasion of the cells.

Acyclovir is a drug blocking the stage of HSV-2 replication in infected cells, while it does not affect the initial stage of viral infection, namely, adsorption and invasion. From this Comparative Example, polyethyleneimine is understood to have a different mechanism of action from that of acyclovir.

**[Table 9]**

| The inhibitory effect of acyclovir on HSV-2 virus invasion of the cells | | | |
|---|---|---|---|
| Sample | Concentration (µg/ml) | Amount of virus invaded in 30 minutes | Amount of virus invaded in 3 hours |
| Acyclovir | 10000 | Toxic | Toxic |
| | 1000 | 68 | Toxic |
| | 100 | 87 | 89 |
| | 10 | 94 | 101 |
| | 1 | 99 | 98 |

| | | | |
|---|---|---|---|
| * Values indicate the amount of virus (%) with setting the amount of virus in the control group with no cyclovir added at 100%. * "Toxic" indicates that the tested cells were killed by the toxicity of the sample. | | | |

### Example 6: Evaluation of the anti-viral activity of polyethyleneimine on various viruses

The anti-viral activity of polyethyleneimine on herpes simplex virus types 1 and 2, measles virus, influenza A virus, and human corona virus were evaluated in vitro.

As the polyethyleneimine, Epomin (registered trademark) SP-003 (a weight average molecular weight of 1,470 as measured by GPC), SP-006 (a weight average molecular weight of 2,310 as measured by GPC), SP-012 (a weight average molecular weight of 3,610 as measured by GPC), SP-018 (a weight average molecular weight of 4,600 as measured by GPC), SP-200 (a weight average molecular weight of 16,560 as measured by GPC), and SP-1000 (a weight average molecular weight of 321,200 as measured by GPC), all manufactured by Nippon Shokubai Co., Ltd., and polyethyleneimine ethoxylate prepared by the below-described procedure (three types including PEIE-1, PEIE-2, and PEIE-3) were used. Hereinbelow, data of SP-003, SP-006, SP-012, SP-018, P-1000, and PEIE-3 will be expressed in the unit of µl/ml, and data of SP-200, PEIE-1, and PEIE-2 will be expressed in the unit of µg/ml.

A test for evaluation of the cytotoxicity of polyethyleneimine in each cell was carried out according to the following procedure.

(1) Cells were cultured in the presence of various concentrations of polyethyleneimine (the concentrations of SP-003, SP-006, SP-012, SP-018, P-1000, and PEIE-3 ranged from 0.002 to 20 µl/ml, and the concentrations of SP-200, PEIE-1, and PEIE-2 ranged from 0.002 to 20 µg/ml) for 72 hours. The cell proliferation count was then measured by trypan blue staining.
(2) Setting the cell count in the control group with no polyethyleneimine added at 100%, the cell count after treatment with each concentration of polyethyleneimine was expressed in %, and the 50% cytotoxic concentration (CC₅₀) was obtained on a graph.
The anti-viral activity of polyethyleneimine on each type of virus was investigated as follows.

### [A] Herpes simplex virus type 1 (HSV-1)

The inhibitory effect of polyethyleneimine on the proliferation of herpes simplex virus type 1 was investigated according to the following procedure.

(1) Vero cells (derived from the kidney of the African green monkey) were detached with trypsin and a solution of 1 × 10⁶ cells/ml was prepared, which was added to a 48-well plate at 200 µl/well.
(2) After one to two days, the cells were confirmed to have grown to a monolayer, and a virus liquid was appropriately diluted with a medium so that a concentration of 0.2 plaque forming unit (PFU)/cell was achieved.
(3) Polyethyleneimine was appropriately diluted with a medium (final concentrations: SP-003, SP-006, SP-012, SP-018, P-1000, and PEIE-3 were 0.0001 to 10 µl/ml, and SP-200, PEIE-1, and PEIE-2 were 0.0001 to 10 µg/ml. These concentrations were similarly applied to the following [B] to [E]).
(4) After removing the medium, viral infection was performed as follows.
   Group A: 25 µl of the virus liquid and 25 µl of polyethyleneimine were simultaneously added to let the infection proceed at room temperature for one hour, while shaking the plate from time to time (approximately once every five to 10 minutes).
   Group B: the same operation as in Group A was performed except for adding a diluted solution (medium) instead of polyethyleneimine of Group A.
(5) After removing the virus liquid, the cells were washed with PBS(-) three times, and 100 µl of polyethyleneimine and 100 µl of the medium were added to each well.
(6) After 24 hours, the cells were transferred to a freezer at -80°C, and the freeze-thaw treatment was performed three times.
(7) Vero cells were cultured so as to form a monolayer in advance in 35 mm dishes, and the sample after completion of three cycles of freeze-thaw was diluted 10° to 10⁵-fold with PBS(-).
(8) The diluted virus liquid was added at 100 µl per dish, and the infection was carried out at 37°C for one hour at room temperature.
(9) The dish was overlaid with 1.5 ml of methyl cellulose (MC)-added MEM medium per dish, and placed in a CO₂ incubator at 37°C.
(10) After one to two days, plaques were confirmed to have grown to a certain size, and then the medium was removed. The cells were fixed and stained with a crystal violet liquid, and the number of plaques was counted under the microscope.
(11) Setting the number of plaques in the control group with no polyethyleneimine added at 100%, the % plaque in the polyethyleneimine-added group was calculated, and a 50% inhibitory concentration for viral proliferation (IC₅₀) was obtained on a semilog graph.
(12) The selectivity index was obtained from the CC₅₀ value and the IC₅₀ value with respect to the Vero cells.
As the herpes simplex virus type 1, an acyclovir (ACV)-sensitive strain (KOS strain) and an acyclovir-resistant strain (A4-3 strain) were used. The inhibitory effect of polyethyleneimine on the proliferation of these viruses is shown in Table 10 (KOS strain) and Table 11 (A4-3 strain). Here, when a selectivity index of 1 or greater is observed, polyethyleneimine is considered to have an anti-viral activity, and particularly, when that of 10 or greater is observed, polyethyleneimine is considered to have a potent anti-viral activity.

SP-006, SP-012, and SP-018 exhibited a high selectivity index with respect to the ACV-sensitive strain in Group A, namely, when they were added simultaneously with the viral infection. Among them, SP-012 was most effective. Very similar results to those observed with the ACV-sensitive strain were obtained with the ACV-resistant strain, and SP-012 was most effective.

**[Table 10]**

| The anti-viral activity on ACV-sensitive HSV-1 (KOS strain) | | | | | |
|---|---|---|---|---|---|
| Sample | Cytotoxicity (CC₅₀) | Anti-viral activity (IC₅₀) | | Selectivity index (CC₅₀/IC₅₀) | |
| | | A | B | A | B |
| SP-006 | 1.1 | 0.013 | >2 | 85 | <1 |
| SP-012 | 0.28 | 0.0026 | >0.5 | 108 | <1 |
| SP-018 | 0.057 | 0.0013 | >0.1 | 44 | <1 |
| SP-200 | 0.0045 | 0.00061 | 0.0057 | 7.4 | 0.79 |
| P-1000 | 0.0044 | 0.00074 | 0.0036 | 5.9 | 1.2 |
| PEIE-1 | >20 | >10 | >10 | ><2 | ><2 |
| PEIE-2 | >20 | >10 | >10 | ><2 | ><2 |
| PEIE-3 | >20 | >10 | >10 | ><2 | ><2 |

| | | | | | |
|---|---|---|---|---|---|
| A: when polyethyleneimine was added to the medium simultaneously with the viral infection B: when polyethyleneimine was added to the medium after the viral infection | | | | | |

**[Table 11]**

| The anti-viral activity on ACV-resistant HSV-1 (A4-3 strain) | | | | | |
|---|---|---|---|---|---|
| Sample | Cytotoxicity (CC₅₀) | Anti-viral activity (IC₅₀) | | Selectivity index (CC₅₀/IC₅₀) | |
| | | A | B | A | B |
| SP-006 | 1.1 | 0.016 | 0.5 | 69 | 2.2 |
| SP-012 | 0.28 | 0.0026 | 0.098 | 108 | 2.9 |
| SP-018 | 0.057 | 0.00095 | 0.053 | 60 | 1.1 |
| SP-200 | 0.0045 | 0.00043 | 0.0032 | 10 | 1.4 |
| P-1000 | 0.0044 | 0.0012 | 0.0082 | 3.7 | 0.54 |
| PEIE-1 | >20 | >10 | >10 | ><2 | ><2 |
| PEIE-2 | >20 | >10 | >10 | ><2 | ><2 |
| PEIE-3 | >20 | >10 | >10 | ><2 | ><2 |

| | | | | | |
|---|---|---|---|---|---|
| A: when polyethyleneimine was added to the medium simultaneously with the viral infection B: when polyethyleneimine was added to the medium after the viral infection | | | | | |

### [B] Herpes simplex virus type 2 (HSV-2)

The inhibitory effect of polyethyleneimine on the proliferation of herpes simplex virus type 2 was investigated by similar plaque assaying to [A]. The results thus obtained are shown in Table 12. SP-006, SP-012, and SP-018 exhibited a high selectivity index in Group A, of which SP-012 was most effective.

**[Table 12]**

| The anti-viral activity on HSV-2 | | | | | |
|---|---|---|---|---|---|
| Sample | Cytotoxicity (CC₅₀) | Anti-viral activity (IC₅₀) | | Selectivity index (CC₅₀/IC₅₀) | |
| | | A | B | A | B |
| SP-003 | 0.071 | 0.0095 | 0.092 | 7.5 | 0.77 |
| SP-006 | 1.1 | 0.035 | 0.77 | 31 | 1.4 |
| SP-012 | 0.28 | 0.0029 | 0.15 | 97 | 1.9 |
| SP-018 | 0.057 | 0.0018 | 0.033 | 32 | 1.7 |
| SP-200 | 0.0045 | 0.0015 | 0.0075 | 3.0 | 0.60 |
| P-1000 | 0.0044 | 0.0021 | 0.0060 | 2.1 | 0.73 |
| PEIE-1 | >20 | >10 | >10 | ><2 | ><2 |
| PEIE-2 | >20 | >10 | >10 | ><2 | ><2 |
| PEIE-3 | >20 | >10 | >10 | ><2 | ><2 |

| | | | | | |
|---|---|---|---|---|---|
| A: when polyethyleneimine was added to the medium simultaneously with the viral infection B: when polyethyleneimine was added to the medium after the viral infection | | | | | |

### [C] Measles virus

The inhibitory effect of polyethyleneimine on the proliferation of measles virus was investigated by similar plaque assaying to [A]. The results thus obtained are shown in Table 13. SP-006 (Group A) exhibited a selectivity index of 10 or greater.

**[Table 13]**

| The anti-viral activity on measles virus | | | | | |
|---|---|---|---|---|---|
| Sample | Cytotoxicity (CC₅₀) | Anti-viral activity (IC₅₀) | | Selectivity index (CC₅₀/IC₅₀) | |
| | | A | B | A | B |
| SP-006 | 1.1 | 0.064 | 0.29 | 17 | 3.8 |
| SP-012 | 0.28 | 0.050 | 0.055 | 5.6 | 5.1 |
| SP-018 | 0.057 | 0.010 | 0.014 | 5.7 | 4.1 |
| SP-200 | 0.0045 | 0.0011 | 0.012 | 4.1 | 0.38 |
| PEIE-1 | >20 | 5.4 | 6.6 | >4 | >3 |
| PEIE-2 | >20 | >10 | >10 | ><2 | ><2 |
| PEIE-3 | >20 | 6.4 | >10 | >3.1 | ><2 |

| | | | | | |
|---|---|---|---|---|---|
| A: when polyethyleneimine was added to the medium simultaneously with the viral infection B: when polyethyleneimine was added to the medium after the viral infection | | | | | |

### [D] Influenza A virus

The inhibitory effect of polyethyleneimine on the proliferation of influenza A virus was investigated according to the following procedure.

(1) MDCK cells (derived from the kidney of the dog) were detached with trypsin and a solution of 1 × 10⁶ cells/ml was prepared, which was added to a 48-well plate at 200 µl/well.
(2) After one to two days, the cells were confirmed to have grown to a monolayer, and a virus liquid was appropriately diluted with a medium so that a concentration of 0.2 PFU/cell was achieved.
(3) Polyethyleneimine was appropriately diluted with a medium.
(4) After removing the medium, viral infection was performed as follows.
   Group A: 25 µl of the virus liquid and 25 µl of polyethyleneimine were simultaneously added to let the infection proceed at room temperature for one hour, while shaking the plate from time to time (approximately once every five to 10 minutes).
   Group B: the same operation as in Group A was performed except for adding a diluted solution (medium) instead of polyethyleneimine of Group A.
(5) After removing the virus liquid, the cells were washed with PBS(-) three times, and 100 µl of polyethyleneimine and 100 µl of a maintenance medium were added to each well.
(6) After 24 hours, the cells were transferred to a freezer at -80°C.
(7) MDCK cells were cultured so as to form a monolayer in advance in 35 mm dishes, and the supernatant of the culture that had been kept frozen was diluted 10⁰ to 10⁵-fold with PBS(-).
(8) The diluted virus liquid was added at 100 µl per dish, and the infection was carried out at 37°C for one hour at room temperature.
(9) The dish was overlaid with 2 ml of an agar medium for influenza virus assay per dish, and after solidification of the agar, the dish was placed in a CO₂ incubator at 37°C.
(10) After two days, plaques were confirmed to have grown to a certain size. After staining, the number of plaques was counted.
(11) Setting the number of plaques in the control group with no polyethyleneimine added at 100%, the % plaque in the polyethyleneimine-added group was calculated, and a 50% inhibitory concentration for viral proliferation (IC₅₀) was obtained on a semilog graph.
(12) The selectivity index was obtained from the CC₅₀ value and the IC₅₀ value with respect to the MDCK cells.
A/NWS/33 (H1N1) virus was used. The results thus obtained are shown in Table 14. Although no polyethyleneimine exhibited a selectivity index of 10 or more, PEIE-3 was most effective, followed by SP-012 and SP-006, both of which exhibited a relatively high selectivity.

**[Table 14]**

| The anti-viral activity on influenza A virus | | | | | |
|---|---|---|---|---|---|
| Sample | Cytotoxicity (CC₅₀) | Anti-viral activity (IC₅₀) | | Selectivity index (CC₅₀/IC₅₀) | |
| | | A | B | A | B |
| SP-003 | 0.057 | 0.026 | 0.046 | 2.2 | 1.2 |
| SP-006 | 0.57 | 0.12 | 0.50 | 4.8 | 1.1 |
| SP-012 | 0.34 | 0.054 | 0.077 | 6.3 | 4.4 |
| SP-018 | 0.064 | 0.061 | 0.062 | 1.0 | 1.0 |
| PEIE-1 | >20 | >10 | >10 | ><2 | ><2 |
| PEIE-2 | >20 | >10 | >10 | ><2 | ><2 |
| PEIE-3 | >20 | 2.2 | 4.5 | >9.1 | >4.4 |

| | | | | | |
|---|---|---|---|---|---|
| A: when polyethyleneimine was added to the medium simultaneously with the viral infection B: when polyethyleneimine was added to the medium after the viral infection | | | | | |

### [E] Human corona virus

The inhibitory effect of polyethyleneimine on the proliferation of human corona virus was investigated according to the following procedure.

(1) MRC-5 cells (derived from the human fetal lung) were detached with trypsin and a solution of 1 × 10⁵ cells/ml was prepared, which was added to a 48-well plate at 300 µl/well.
(2) After one to two days, the cells were confirmed to have nearly grown to a monolayer, and a virus liquid was appropriately diluted with a medium so that a concentration of 0.002 TCID₅₀ was achieved.
(3) Polyethyleneimine was appropriately diluted with a medium.
(4) After removing the medium, viral infection was performed as follows.
   Group A: 25 µl of the virus liquid and 25 µl of polyethyleneimine were simultaneously added to let the infection proceed at room temperature for one hour, while shaking the plate from time to time (approximately once every five to 10 minutes).
   Group B: the same operation as in Group A was performed except for adding a diluted solution (medium) instead of polyethyleneimine of Group A.
(5) After removing the virus liquid, the cells were washed with PBS(-) three times, and 150 µl of polyethyleneimine and 150 µl of a medium were added to each well, and the cells were treated at 36°C.
(6) After three days, the cytopathic effect (CPE) (rounding and sloughing) were observed (and the degree of the effect was recorded as needed), and the cells were then transferred to a freezer at -80°C.
(7) In a 96-well plate, MRC-5 cells were nearly grown to a monolayer.
(8) A virus liquid that had been subjected to three cycles of freeze-thaw was used to prepare a 3-fold dilution series (3¹ to 3¹⁰) with a medium. The resulting dilution series were added to the above plate, four wells per dilution, 25 µl per well, to perform the infection (at room temperature for one hour).
(9) The medium was added at 100 µl/well, and the plate was placed in a CO₂ incubator at 36°C.
(10) After five days, the presence or absence of CPE was checked.
(11) TCID₅₀ was obtained by the Reed-Muench method.
(12) The selectivity index was obtained from the CC₅₀ value and the TCID₅₀ value with respect to the MRC-5 cells.
The results thus obtained are shown in Table 15. Nearly all of the polyethyleneimine exhibited a high selectivity index, of which SP-012 was most effective. PEIE-3 also exhibited a high selectivity. In this experiment also, a higher selectivity index was observed in Group A than in Group B.

**[Table 15]**

| The anti-viral activity on human corona virus | | | | | |
|---|---|---|---|---|---|
| Sample | Cytotoxicity (CC₅₀) | Anti-viral activity (IC₅₀) | | Selectivity index (CC₅₀/IC₅₀) | |
| | | A | B | A | B |
| SP-003 | 0.14 | 0.011 | 0.011 | 13 | 13 |
| SP-006 | 1.2 | 0.014 | 0.16 | 86 | 7.5 |
| SP-012 | 0.37 | 0.0016 | 0.0092 | 230 | 40 |
| SP-018 | 0.13 | 0.0019 | 0.0026 | 68 | 50 |
| SP-200 | 0.0052 | 0.00029 | 0.00048 | 18 | 11 |
| P-1000 | 0.0060 | 0.00058 | 0.0014 | 10 | 4.3 |
| PEIE-1 | >20 | 3.7 | 4.3 | >5 | >5 |
| PEIE-2 | >20 | 3.2 | 3.3 | >6 | >6 |
| PEIE-3 | >20 | 0.29 | 1.2 | >69 | >17 |

| | | | | | |
|---|---|---|---|---|---|
| A: when polyethyleneimine was added to the medium simultaneously with the viral infection B: when polyethyleneimine was added to the medium after the viral infection | | | | | |

### [Preparation of polyethyleneimine ethoxylate (pete-1, PEIE-2, and PEIE-3)]

### (a) PEIE-1

In a 1.2 L autoclave, 172.3 g of polyethyleneimine (Epomin SP-006 manufactured by Nippon Shokubai Co., Ltd.) was placed, and the temperature was raised to 150°C. Subsequently, 167.4g (3.8 mol) of ethylene oxide was fed to add ethylene oxide to polyethyleneimine. The temperature was then cooled to 80°C, and 2.5 g of a 49% aqueous solution of KOH was added, and the temperature was raised to 165°C. While bubbling nitrogen at a flow rate of 20 ml/minute, the pressure was reduced to 0.05 MPa to dehydrate the mixture. Subsequently, 443.5 g (10.1 mol) of ethylene oxide was fed to add ethylene oxide to polyethyleneimine. The temperature was then cooled to 80°C, and acetic acid was added to adjust pH to 7. Then, 10% of purified water with respect to the total amount of polyethyleneimine polyethoxylate was added, and the pressure was reduced to 0.005 MPa while bubbling nitrogen at a flow rate of 20 ml/minute, whereby deaeration was performed for five hours. Then, light impurities were removed to give the objective polyethyleneimine polyethoxylate (PEIE-1), which had a weight ratio of PEI / PEG = 22 / 78, a weight average molecular weight of 5,500 as measured by GPC, and a hydroxyl value of 370 ± 20 mg KOH/g as measured by the phthalic anhydride method.

### (b) PEIE-2

In a 1.2L autoclave, 172.3 g of polyethyleneimine (Epomin SP-018 manufactured by Nippon Shokubai Co., Ltd.) was placed, and the temperature was raised to 150°C. Subsequently, 167.4g (3.8 mol) of ethylene oxide was fed to add ethylene oxide to polyethyleneimine. The temperature was then cooled to 80°C, and 2.5 g of a 49% aqueous solution of KOH was added, and the temperature was raised to 165°C. While bubbling nitrogen at a flow rate of 20 ml/minute, the pressure was reduced to 0.05 MPa to dehydrate the mixture. Subsequently, 349.5 g (7.9 mol) of ethylene oxide was fed to add ethylene oxide to polyethyleneimine. The temperature was then cooled to 80°C, and acetic acid was added to adjust pH to 7. Then, 10% of purified water with respect to the total amount of polyethyleneimine polyethoxylate was added, and the pressure was reduced to 0.005 MPa while bubbling nitrogen at a flow rate of 20 ml/minute, whereby deaeration was performed for five hours. Then, light impurities were removed to give the objective polyethyleneimine polyethoxylate (PEIE-2), which had a weight ratio of PEI / PEG = 25 / 75, a weight average molecular weight of 7,500 as measured by GPC, and a hydroxyl value of 345 ± 20 mg KOH/g as measured by the phthalic anhydride method.

### (c) PEIE-3

In a 1.2L autoclave, 180.0 g of polyethyleneimine (Epomin SP-006 manufactured by Nippon Shokubai Co., Ltd.) was placed, and the temperature was raised to 150°C. Subsequently, 174.8 g (3.97 mol) of ethylene oxide was fed to add ethylene oxide to polyethyleneimine. The temperature was then cooled to 80°C, and 28.8 g of a 49% aqueous solution of KOH was added, and the temperature was raised to 165°C. While bubbling nitrogen at a flow rate of 20 ml/minute, the pressure was reduced to 0.05 MPa to dehydrate the mixture. Subsequently, 469.4 g (10.67 mol) of ethylene oxide was fed to add ethylene oxide to polyethyleneimine. The temperature was then cooled to 80°C, and the solution in the autoclave was released so that the amount of remaining solution after releasing was 173.0 g. Subsequently, 620.1 g (13.2 mol) of ethylene oxide was fed to add ethylene oxide to polyethyleneimine. The temperature was then cooled to 80°C and acetic acid was added to adjust pH to 7. Then, 10% of purified water with respect to the total amount of polyethyleneimine polyethoxylate was added, and the pressure was reduced to 0.005 MPa while bubbling nitrogen at a flow rate of 20 ml/minute, whereby deaeration was performed for five hours. Then, light impurities were removed and 20% of purified water with respect to the total amount of the product was further added to give the objective polyethyleneimine ethoxylate (PEIE-3) as an aqueous solution having an 80% solid content. This PEIE-3 had a weight ratio of PEI / PEG = 5 / 95, a weight average molecular weight of 10,000 as measured by GPC, and a hydroxyl value of 74.0 ± 3.0 mg KOH/g as measured by the phthalic anhydride method.

### Example 7: The preventive effect of external application of polyethyleneimine (SP-006 and SP-012) on the development of the disease in HSV-2-infected animals

An infection experiment was conducted again to examine the anti-viral action of polyethyleneimine exhibiting a high selectivity index in the in vitro anti-HSV-2 activity test in Example 6, namely SP-006 and SP-012, in mice.

Five to six-week-old female BALB/c mice were locally (genital) inoculated with HSV-2 in an amount of 20 µl (containing 1 × 10⁵ PFU of virus) per mouse. SP-006, SP-012, or phosphate-buffered physiological saline were administered by application to the genital mucosa in a dose of 0.2 mg or 0.02 mg per administration (20 µl) (concentration: 10 mg/ml or 1 mg/ml) for a total of five times, namely one hour before the viral infection, immediately after, six hours after, 24 hours after, and 48 hours after the infection. No toxicity was observed in the mice under these administration conditions. The results thus obtained are shown in Table 16.

In the control group, four out of five mice died within nine days of infection (the death rate of 80%). The death rates were 20 to 60% and 0 to 80% in the SP-006-administration group and the SP-012-administration group, respectively. In a group administered with high dose of SP-012 (0.2 mg each time), all of the five mice did not develop genital herpes at all.

Three days after the HSV-2 infection, the genital of the mice was washed with PBS, and the amount of virus therein was measured by plaque assaying. As a result, the amount of virus was dose-dependently suppressed by administration of SP-006 and SP-012. Comparing at the same dose, SP-012 exhibited a higher effect than did SP-006.

**[Table 16]**

| The therapeutic effect of SP-006 and SP-012 in HSV-2-infected animals | | | | |
|---|---|---|---|---|
| Sample | PEI dose per administration | Amount of virus 3 days after infection (x10²PFU/100µl) | Survival rate (%) | Days until death (median number of days) |
| Control (PBS) | - | 87 ± 59 | 1/5 (20) | 7,8,9,9 (8.5 ± 0.75) |
| SP-006 | 0.2 mg | 28 ± 24 | 4/5 (80) | 10 (10.0 ± 0) |
| SP-006 | 0.02 mg | 48 ± 25 | 2/5 (40) | 8,9 (8.5 ± 0.50) |
| SP-012 | 0.2 mg | 2.5 ± 2.3 | 5/5 (100) | - |
| SP-012 | 0.02 mg | 13 ± 19 | 1/5 (20) | 9 (90±0) |

| | | | | |
|---|---|---|---|---|
| * The number of mice was five in all cases | | | | |

### Example 8: Verification that polyethyleneimine (SP-012) does not give rise to drug-resistant virus

Whether or not herpes simplex virus type 2 (HSV-2) acquires drug resistance with administration of polyethyleneimine (SP-012) was verified.

HSV-2-infected Vero cells were subcultured three times in the presence of 100 µg/ml of SP-012, and then seven times in a concentration of 200 µg/ml. Subsequently, 10 clones of surviving viruses were separated by plaque assaying. The 10 clones thus separated and the original virus strain were tested for the sensitivity to SP-012. The sensitivity was expressed as a concentration of SP-012 at which the viral proliferation was inhibited by 50% (IC₅₀). The results thus obtained are shown in Table 17. As shown in Table 17, all of the 10 clones exhibited approximately the same IC₅₀ as the original virus strain (wild strain) did, showing that the sensitivity to SP-012 was maintained. That is, it was judged that the viruses did not acquire resistance. From these results, it was speculated that it would be highly unlikely that long-term administration of SP-012 to the living body causes a problem such as interruption of administration due to the emergence of drug-resistant virus.

**[Table 17]**

| Virus strain | IC₅₀ (mg/ml) |
|---|---|
| Wild strain | 4.7 |
| Clone 1 | 4.5 |
| Clone 2 | 2.4 |
| Clone 3 | 2.3 |
| Clone 4 | 3.3 |
| Clone 5 | 3.3 |
| Clone 6 | 1.6 |
| Clone 7 | 2.4 |
| Clone 8 | 4.9 |
| Clone 9 | 2.3 |
| Clone 10 | 2.3 |

### Example 9: Comparison of effects between polyethyleneimine (SP-012) and acyclovir in HSV-2-infected animals

The time courses of HSV-2 proliferation in the genital of the mice treated with the same doses of acyclovir, a therapeutic agent for herpes simplex virus type 2 (HSV-2), and polyethyleneimine (SP-012) was traced.

Five to six-week-old female BALB/c mice were locally (genital) inoculated with HSV-2 in an amount of 20 µl (containing a reduced viral amount of 1 × 10⁴ PFU per mouse in consideration of tracing also untreated control mice). SP-012 and acyclovir, in an amount of 0.2 mg each per administration, or phosphate-buffered physiological saline were administered to the genial one hour before, one hour after, and eight hours after the viral infection, and from the next day, twice daily (8:00 am and 6:00 pm) until seven days after the viral infection. From the first day to 12 days after the viral infection, the genital was washed with phosphate-buffered physiological saline every day, and the amount of virus therein was measured in each mouse. Also, for 15 days, the severity of the development of the disease and the case of death were recorded.

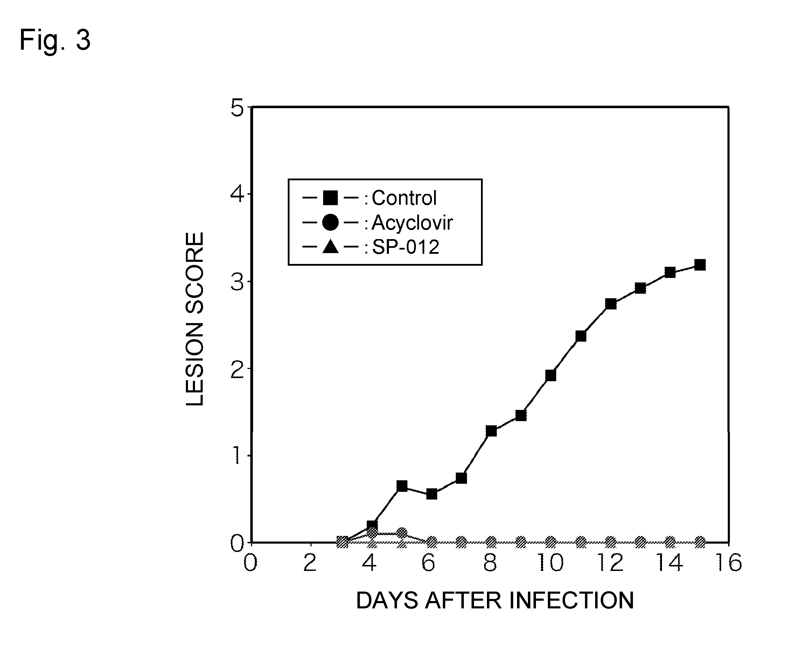

As shown in Table 18 and Figure 3, all of the 11 mice in the control group developed genital inflammation caused by the HSV-2 infection. Also, the virus was detected in all of the mice, of which seven died (the death rate of 64%). In the acyclovir-administration group, the virus was detected in one out of 10 mice, which exhibited transient inflammation. No mice died in the acyclovir-administration group. In contrast, in the SP-012-administration group, none of the 10 mice developed the disease, and the virus was not found in any of them. No mice died in the SP-012-administration group. From the above results, SP-012 is found to be as effective as or more effective in the treatment of genital herpes in comparison with the same dose of acyclovir.

**[Table 18]**

| Sample | Number of death/total number | Days until death | Number of HSV-2 positive mouse/total number |
|---|---|---|---|
| Control | 7/ 11 | 9, 9, 11, 12, 14, 14,15 | 11/11 |
| Acyclovir | 0/ 10 | - | 1/10 |
| SP-012 | 0/ 10 | - | 0/10 |

### Example 10: Evaluation of the anti-viral activity of a complex of polyethyleneimine (SP-012) and liposomes on herpes simplex virus type 2 (HSV-2)

A complex of polyethyleneimine (SP-012) and liposomes was prepared, and the anti-viral activity of this complex on herpes simplex virus type 2 (HSV-2) was evaluated in vitro.

### (1) Preparation of a polyethyleneimine-neutral liposome complex

Neutral liposomes (egg yolk lecithin (EPC) : cholesterol (Cho) = 5 : 5, molar ratio) was mixed with an aqueous solution of polyethyleneimine (SP-012) (the final lipid concentration of 20 mg/ml and polyethyleneimine concentration of 100 mg/ml), and the pH was adjusted to 7.0 to 7.4 with 1N hydrochloric acid.

### (2) Preparation of a polyethyleneimine-PEG liposome complex

Liposomes coated with polyethylene glycol (PEG) (EPC : Cho : PEG = 5 : 5 : 0.5, molar ratio) were mixed with an aqueous solution of polyethyleneimine (SP-012) (the final lipid concentration of 20 mg/ml and polyethyleneimine concentration of 100 mg/ml), and the pH was adjusted to 7.0 to 7.4 with 1N hydrochloric acid.

### (3) Evaluation of the anti-viral activity

Following the same procedure as in Example 6 (particularly, see [B]), the cytotoxicity (50% cytotoxic concentration (CC₅₀)), the anti-viral activity on herpes simplex virus type 2 (50% inhibitory concentration for viral proliferation (IC₅₀)), and the selectivity index (CC₅₀/IC₅₀) of a sample of each of acyclovir, polyethyleneimine (SP-012), a polyethyleneimine (SP-012)-neutral liposome complex, and a polyethyleneimine (SP-012)-PEG liposome complex were obtained. The concentration of acyclovir or polyethyleneimine ranged from 0.02 to 2000 µg/ml in the cytotoxicity test, or from 0.01 to 1000 µg/ml in the anti-viral activity test. The results thus obtained are shown in Table 19. A polyethyleneimine-neutral liposome complex or a polyethyleneimine-PEG liposome complex exhibited a higher selectivity index than did polyethyleneimine alone, showing that the polyethyleneimine-neutral liposome complex or the polyethyleneimine-PEG liposome complex has a remarkably high anti-viral activity.

**[Table 19]**

| Sample | Cytotoxicity (CC₅₀) (µg/ml) | Anti-viral activity (IC₅₀)(µg/ml) | | Selectivity index (CC₅₀/IC₅₀) | |
|---|---|---|---|---|---|
| | | A | B | A | B |
| Acyclovir | 890 | 0.91 | 0.92 | 980 | 970 |
| PEI (SP-012) | 260 | 3.1 | 170 | 84 | 1.5 |
| PEI (SP-012) + neutral liposome | 160 | 0.25 | 110 | 640 | 1.5 |
| PEI (SP-012) + PEG liposome | 170 | 0.47 | 63 | 360 | 2.7 |

| | | | | | |
|---|---|---|---|---|---|
| A: when the sample was added to the medium simultaneously with the viral infection B: when the sample was added to the medium after the viral infection | | | | | |

All of the publications, patents, and patent applications cited in the present specification are hereby incorporated by reference in their entirety.

## Claims

1. A pharmaceutical composition for treating or preventing a viral infection, comprising polyalkyleneimine having a weight average molecular weight ranging from 300 to 400,000.

2. The pharmaceutical composition according to Claim 1, wherein the pharmaceutical composition is an external pharmaceutical composition comprising 20 mg/ml or less of the polyalkyleneimine.

3. The pharmaceutical composition according to Claim 1 or 2, wherein the polyalkyleneimine is linear or branched polyethyleneimine.

4. The pharmaceutical composition according to any of Claims 1 to 3, further comprising a lipid particle.

5. The pharmaceutical composition according to any of Claims 1 to 4, which is for treating or preventing an infection caused by an enveloped virus.

6. The pharmaceutical composition according to any of Claims 1 to 4, which is for treating or preventing a herpes virus infection.

7. A virus-inactivating agent for administration to a living body, comprising polyalkyleneimine.

8. An inhibitor of viral adsorption to a cell, comprising polyalkyleneimine.

9. An inhibitor of viral invasion of a cell, comprising polyalkyleneimine.
